# EUROPÄISCHE PATENTSCHRIFT

(11) **EP 0 946 952 B1**
(45) Veröffentlichungstag und Bekanntmachung des Hinweises auf die Patenterteilung: **25.04.2001**
(21) Anmeldenummer: 97912165.4
(22) Anmeldetag: 10.10.1997
(51) Int. Cl.: H01C 7/02, A61F 7/02, H05B 3/34

(54) **HEIZDECKE**
ELECTRIC BLANKET
COUVERTURE CHAUFFANTE

(30) Priorität: 11.10.1996 DE 19642037
(43) Veröffentlichungstag der Anmeldung: 06.10.1999
(73) Patentinhaber: Thermamed GmbH, 59065 Hamm (DE)
(72) Erfinder: GENTNER, Nikolaus, D-81243 München (DE); KREINER, Hans-Jürg, D-81545 München (DE); KREINER, Jürg, A-5020 Salzburg (AT)
(74) Vertreter: Möbus, Steffen
(86) Internationale Anmeldenummer: EP9705600
(87) Internationale Veröffentlichungsnummer: WO9816938

(56) Entgegenhaltungen:
- DE-A- 3 907 557
- DE-A- 4 233 118
- FR-A- 2 263 658
- GB-A- 2 285 729
- US-A- 4 186 294
- US-A- 5 422 462

## Beschreibung

Die Erfindung betrifft eine Heizdecke mit einer flächigen elektrisch bezeizbaren Widerstandschicht, die zwischen außen liegenden elektrisch nicht leitenden Schichten angeordnet ist.

Eine derartige Heizdecke ist beispielsweise aus der DE 35 24 631 A1 bekannt. Bei der bekannten Heizdecke wird als elektrisch betreibbare Widerstandsheizschicht ein aus einem Copolymerisat bestehender Film, dem elektrisch leitender Kohlenstoff zugemischt ist, verwendet. Eine mit einer derartigen Widerstandsheizschicht versehene Heizdecke ist zwar flexibler als herkömmliche Heizdecken, die mit Widerstandsheizdrähten beheizt werden, jedoch ist durch den Copolymerisatfilm bei weitem noch nicht die Flexibilität erreicht, welche ein zur Anpassung an den Körper einer Person erforderlich ist. textiles Gewebe hat.

FR-A-2 263 658 offenbart eine Heizdecke, welche Heizdeckenelemente aufweist. Die Heideckenelemente sind um eine Querleiste umklappbar.

Aufgabe der Erfindung ist es daher, eine Heizdecke der eingangs genannten Art zu schaffen, welche eine extrem hohe Flexibilität hat.

Diese Aufgabe wird erfindungsgemäß dadurch gelöst, daß die Widerstandsheizschicht eine flexible, im wesentlichen nur aus Kohlenstoffasern (Kohlefasern) gebildete Schicht aufweist. In bevorzugter Weise sind die Kohlefasern der Heizschicht zu einem flächigen Textilerzeugnis, insbesondere aus Garnen gebildeten Gewebe, verarbeitet. Das Kettfadensystem des Gewebes besteht aus Garnen mit Kohlefasern, die eine größere Faserlänge aufweisen als die Garne, welche zur Bildung des Schußfadensystems dienen. In bevorzugter Weise können die Fäden des Kettfadensystems aus Zwirnen bestehen, während die Fäden des Schußfadensystems aus einfachen Garnen gebildet sind. Die im wesentlichen nur aus Kohlefasern bestehende Heizschicht enthält etwa 97 % Kohlefasern und 3 % Stabilisatoren. Die Kohlefasern können aus Hydrat-Zellulose hergestellt sein.

Im Gegensatz zu herkömmlichen Heizsystemen in Heizdecken, insbesondere solchen mit Drahtheizungssystemen, reicht als Versorgungsspannung 24 Volt und weniger aus. Bevorzugt kommt eine Spannungsquelle mit 12 Volt zur Anwendung.

Die erfingungsgemäße Heizdecke kommt bevorzugt im medizinischen Bereich zur Anwendung. Sowohl im stationären als auch im ambulanten Bereich kann sie hier zum Einsatz kommen. Da die Widerstandsheizschicht, welche praktisch nur aus Kohlefasern gebildet ist, eine äußerst hohe Flexibilität aufweist, welche der von aus Wolle hergestelltem Gewebe vergleichbar ist, kann die Heizdecke zur Aufrechterhaltung der Körpertemperatur von Patienten, beispielsweise von Unfallopfern oder auch von Patienten während einer Operation verwendet werden. Daß die meisten Patienten während einer Operation frösteln, ist seit langem bekannt. Ursache hierfür ist nicht nur die fehlende Bekleidung und die niedrige Raumtemperatur, sondern auch die Anästhesie. Die Körpertemperatur kann dabei erheblich absinken. Bei längeren Operationen, beispielsweise nach vier Stunden, sind die Patienten nachweisbar unterkühlt. Dies ist auch der Grund, weshalb bei diesen Patienten sehr häufig Infektionen auftreten. Die erforderliche Erholungsphase nach der Operation verlängert sich daher erheblich. Überall dort, wo Unterkühlung (Hypothermie) des Patienten ein Problem darstellt, ist die Kohlefaserheizdecke einsetzbar, beispielsweise bei Unfallrettung, Katastrophenschutz, Bergrettung und dergl.

In bevorzugter Weise bildet die aus den Kohlefasern gebildete Heizschicht einen Kern der Decke, der nach oben hin mit einer dünnen Isolationsauflage zur Verringerung der Wärmeabgabe nach oben versehen sein kann. Dieser Kern kann von einem Isoliermantel, beispielsweise aus Nylon oder einer Silikonbeschichtung umgeben sein.

Die Decke kann quer zu ihrer Längsausdehnung in mehrere Deckenelemente mit zugeordneten Heizschichtelementen unterteilt sein. Die einzelnen quer verlaufenden Dekkenelemente können durch eine Längsleiste an einer Seite miteinander verbunden sein. Jeweilige Deckenelemente können um die Längsleiste weggeklappt werden, so daß ein zu behandelndes Körperteil des unter der Decke liegenden Patienten freigelegt werden kann. Das Kohlefasergewebe ist bevorzugt in der Weise in den Deckenelementen angeordnet, daß das Kettfadensystem quer zur Längsausdehnung der Decke, d.h. in Längsrichtung des jeweiligen streifenförmigen Deckenelements, im wesentlichen ausgerichtet ist.

Das Kohlefasergewebe ist unempfindlich gegen Stichversetzungen und andere Verletzungen sowie auch gegen ein direktes Eindringen von Feuchtigkeit oder Wasser. Das Gewebe besitzt vor allem in Richtung des Kettfadensystems eine extrem hohe Reißfestigkeit. Durch in den jeweiligen Deckenelementen zum Einsatz kommenden Thermofühlern wird eine Überhitzung vermieden.

Neben dem medizinischen Einsatz kann die Decke auch in ungeteilter Form als durchgehende Wärmedecke verwendet werden. Bevorzugt kann die Heizdecke auch zum Heizen von Fahrzeugsitzen, insbesondere Kraftfahrzeugsitzen, verwendet werden. Wegen ihrer geringen Dicke kann sie in die äußere Lage der Sitzpolsterung integriert sein. Sie kann jedoch auch in einen Sitzüberzug integriert sein, der auswechselbar am Fahrzeugsitz angebracht werden kann.

Anhand der Figuren wird an einem Ausführungsbeispiel die Erfindung noch näher erläutert.
- Fig. 1: zeigt eine Draufsicht auf ein Ausführungsbeispiel der Decke;
- Fig. 2: zeigt eine schnittbildliche Darstellung durch ein Deckenelement; und

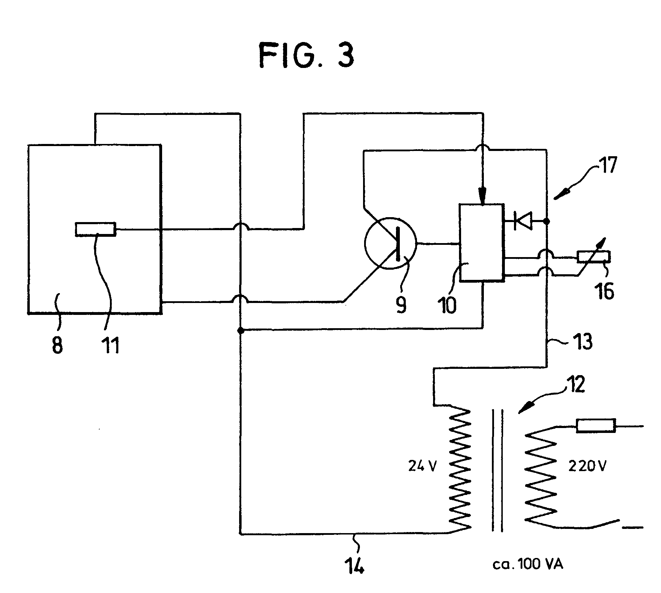

- Fig. 3: zeigt eine Schaltungsanordnung zur geregelten Stromversorgung eines Heizelementes.

Eine in den Figuren als Ausführungsbeispiel dargestellte Heizdecke besteht aus Heizdeckenelementen 2, die über eine seitliche Längsleiste 3 miteinander verbunden sind. Die Heizdeckenelemente 2 sind quer zur Längsausdehnung der Heizdecke 1 voneinander getrennt, so daß sie wahlweise um die Längsleiste 3 weggeklappt werden können. Auf diese Weise kann ein gewünschtes Körperteil eines unter der Heizdecke liegenden Patienten freigelegt werden.

In Längsrichtung der Heizdecke kann jedes Heizelement eine Ausdehnung von etwa 15 cm bis 25 cm aufweisen. In der Breite der Heizdecke kann jedes Heizelement eine Abmessung von etwa 60 cm oder mehr aufweisen.

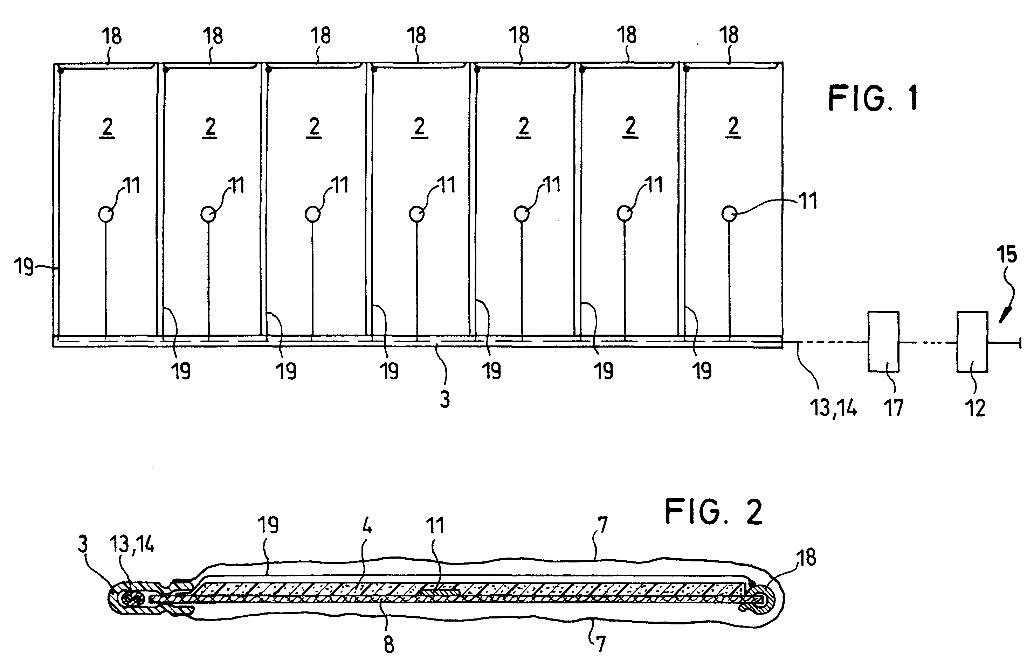

Jedes Heizdeckenelement 2 besitzt eine aus Kohlefasern gebildete Heizschicht. In bevorzugter Weise sind die Kohlefasern zu Garnen verarbeitet. die ein Gewebe bilden. Die Dicke des Gewebes beträgt ca. 1 bis 2 mm und hat die gleiche Flexibilität wie ein aus Wollfasern bestehendes Gewebe. Die Kettfäden des Kohlefasergewebes sind in der Fig. 1 in den jeweiligen Heizdeckenelementen 2 im wesentlichen parallel zu den jeweiligen Trennlinien zwischen den Heizdeckenelementen 2 ausgerichtet. An der Oberseite besitzt das Heizschichtelement 8 eine Wärmeisolierschicht oder Wärmedämmschicht 4. Diese verhindert eine Abstrahlung der Wärme nach einer Oberflächenseite hin. Hierdurch wird gewährleistet. daß eine Abstrahlung von Wärme von der einen Oberfläche, welche dem Patientenkörper abgewandt ist, verhindert wird. Gegebenenfalls kann der aus Isolierschicht 4 und Heizschicht 8 bestehende Verbundkörper an seiner Oberseite und an seiner Unterseite mit einer dünnen Silikonschicht versehen sein. Der Verbundkörper wird bevorzugt noch von einem Überzug 7 umgeben, der aus Nylon, Papier oder einem anderen geeigneten Material bestehen kann.

Die Stromversorgung der einzelnen Heizdeckenelemente 2 kann über in der Längsleiste 3 angeordneten Stromversorgungsleitungen 13, 14 von einer zentralen Stromversorgung 15 aus erfolgen. Hierbei genügt eine Versorgungsspannung von etwa 12 Volt, z.B. eine Fahrzeugbatterie beim Einsatz an einem Verkehrsunfallort. Der Anschluß kann über Streifenkontakte 18 und eine elektrische Rückführleitung 19 erfolgen.

Zur Regelung der Stromversorgung in Abhängigkeit von einer voreinstellbaren Temperatur ist für jedes Heizdeckenelement eine separate Stromversorgung vorgesehen. Hierzu kann in der zentralen Stromversorgungseinrichtung 15 ein Netzgerät 12 vorgesehen sein, welches von der Netzspannung auf die Versorgungsspannung von ca. 24 Volt Gleichspannung für das jeweilige Heizschichtelement 8 und die zugeordnete Temperatursteuerung 17 transformiert.

In jedem Heizdeckenelement 2 ist wenigstens ein Temperaturfühler 11 vorgesehen. Der Temperaturfühler 11 ist mit einem Vergleicher 10 verbunden, dem über eine Einstellvorrichtung 16 ein Temperatursollwert, beispielsweise Körpertemperatur (37°C), als Solltemperatur eingebbar ist. Die vom Temperaturfühler 11 erfaßte Temperatur wird mit der vorgegebenen Temperatur im Vergleicher 10 verglichen, und in Abhängigkeit vom Vergleichsergebnis wird ein Stellelement 9 für den Versorgungsstrom, welcher dem Heizschichtelement 8 zugeführt wird, angesteuert. Bei dem Stellelement 9 kann es sich um eine Einstelleinrichtung für die Stromstärke oder auch um einen Ein/Aus-Schalter handeln.

Die Kohlefaserschicht besitzt pro 1 cm 2 Oberfläche einen Widerstandswert von ca. 1 Ω. Die für die Heizdecke benötigte Gesamtleistung beträgt maximal etwa 300 Watt. Die Leistung kann auch auf geringere Werte geregelt werden. Die Heizdecke kann aus neun Querstreifen bestehen, von denen jeder Streifen eine Fläche von etwa 190 mm x 600 mm aufweist. Die Gesamtfläche der Heizdecke kann etwa 1700 mm x 600 mm betragen. Der Temperatureinstellbereich beträgt 15°C bis 40°C. Die Heizdecke kann jedoch auch nur die Abmessungen eines Kissens aufweisen.

## Patentansprüche

1. Heizdecke mit einer elektrisch betreibbaren Widerstandsheizschicht, die im wesentlichen nur aus Kohlefasern, die zu einem flächigen, flexiblen Fasergewebe verarbeitet sind, gebildet ist,
**dadurch gekennzeichnet,**
daß die Heizdecke (1) quer zu ihrer Längsausdehnung in mehrere Heizdeckenelemente (2) unterteilt ist, die jeweils ein zwischen elektrisch nicht-leitenden, flexiblen Schichten angeordnetes Heizschichtelement (8) aufweisen, und wobei die quer verlaufenden Heizdeckenelemente (2) seitlich durch eine Längsleiste (3) an einer einzigen Seite miteinander verbunden und einzeln um die Längsleiste (3) wegklappbar sind.

2. Heizdecke nach Anspruch 1, dadurch gekennzeichnet, daß ein Kett- und Schußfadensystem aufweisedes Gewebe aus von den Kohlefasern gebildeten Garnen die Heizschichtelemente (8) bildet.

3. Heizdecke nach Anspruch 2, dadurch gekennzeichnet, daß das Kettfadensystem des Gewebes aus Zwirnen besteht.

4. Heizdecke nach Anspruch 2 oder 3, dadurch gekennzeichnet, daß die Faserlänge der Garne des Kettfadensystems länger bemessen ist als die Faserlänge der Garne des Schußfadensystems.

5. Heizdecke nach einem der Ansprüche 2 bis 4, dadurch gekennzeichnet, daß das Schußfadensystem aus einfachen Garnen gebildet ist.

6. Heizdecke nach einem der Ansprüche 1 bis 5, dadurch gekennzeichnet, daß das Kettfadensystem des Kohlefasergewebes im wesentlichen quer zur Längsausdehnung der Heizdecke (1) ausgerichtet ist.

7. Heizdecke nach einem der Ansprüche 1 bis 6, dadurch gekennzeichnet, daß die elektrisch nicht-leitenden, flexiblen Schichten aus Silikon bestehen.

8. Heizdecke nach einem der Ansprüche 1 bis 7, dadurch gekennzeichnet, daß jedes Heizschichtelement (8) mit einer zugeordneten Stromversorgungseinrichtung verbunden ist.

9. Heizdecke nach Anspruch 8, dadurch gekennzeichnet, daß für jedes Heizschichtelement (8) ein Temperaturfühler (11) mit zugeordnetem Temperaturregler, der den Versorgungsstrom steuert, vorgesehen ist.

10. Heizdecke nach einem der Ansprüche 1 bis 9, dadurch gekennzeichnet, daß jedes Heizschichtelement (8) auf seiner Oberseite eine Wärmeisolierschicht (4) aufweist.

11. Heizdecke nach einem der Ansprüche 1 bis 10, dadurch gekennzeichnet, daß die Versorgungsspannung für die Heizschicht 24 Volt oder geringer ist und insbesondere ca. 12 Volt beträgt.

12. Heizdecke nach einem der Ansprüche 1 bis 11, dadurch gekennzeichnet, daß eine jeweilige Umhüllung oder Überzug (7) für die Heizschicht auswechselbar ist.

## Claims

1. An electric blanket with an electrically operable resistance heating layer which is made from a sheetlike flexible woven fabric essentially comprising only carbon fibres,
**characterised in that**
across is longitudinal extent the electric blanket (1) is divided into several electric blanket elements (2) each comprising a heating element layer (8) arranged between electrically non-conductive flexible layers, and with the electric blanket elements (2) extending across, being interconnected on one side by a longitudinal strip (3) so as to be individually hingeable on the longitudinal strip (3).

2. The electric blanket according to claim 1, characterised in that the heating element layer (8) is a woven material with a warp thread system and a weft thread system made of yarns comprising carbon fibres.

3. The electric blanket according to claim 2, characterised in that the warp thread system of the woven material comprises twisted yarns.

4. The electric blanket according to claim 2 or 3, characterised in that the fibres of the yarns of the warp thread system are longer than the fibres of the yarns of the weft thread system.

5. The electric blanket according to one of claims 2 to 4, characterised in that the weft thread system comprises single yarns.

6. The electric blanket according to one of claims 1 to 5, characterised in that the warp thread system of the carbon fibre woven fabric is essentially aligned across the longitudinal extent of the electric blanket (1).

7. The electric blanket according to one of claims 1 to 6, characterised in that the electrically non-conductive flexible layers are made of silicon.

8. The electric blanket according to one of claims 1 to 7, characterised in that each heating element layer (8) is connected to an associated power supply device.

9. The electric blanket according to claim 8, characterised in that for each heating element layer (8) a temperature sensor (11) is provided with an associated temperature controller which controls the supply current.

10. The electric blanket according to one of claims 1 to 9, characterised in that on its top each heating element layer (8) comprises a heat insulation layer (4).

11. The electric blanket according to one of claims 1 to 10, characterised in that the supply voltage for the heating layer is 24 volt or less, in particular approx. 12 volt.

12. The electric blanket according to one of claims 1 to 11, characterised in that the respective shell or cover (7) of the heating layer is exchangeable.

## Revendications

1. Couverture chauffante comprenant une couche chauffante rhéostatique ne se composant, en substance, que de fibres de carbone qui sont travaillées pour former un tissu de fibres bidimensionnel flexible, caractérisée en ce que la couverture chauffante (1) est partagée transversalement à sa longueur en plusieurs éléments de couverture chauffante (2) qui présentent chacun un élément de couche chauffante (8) placé entre des couches flexibles non conductrices d'électricité, les éléments de couverture chauffante (2) orientés transversalement étant reliés entre eux d'un seul côté par une lisière de renforcement longitudinale (3) et pouvant être rabattus individuellement autour de la lisière de renforcement longitudinale (3).

2. Couverture chauffante selon la revendication 1, caractérisée en ce que les éléments de couche chauffante (8) sont formés d'un tissu présentant un système de chaîne et de trame de fils à base de fibres de carbone.

3. Couverture chauffante selon la revendication 2, caractérisée en ce que le système de fils de chaîne du tissu se compose de fils retors.

4. Couverture chauffante selon la revendication 2 ou 3, caractérisée en ce que la longueur de fibres des fils du système de fils de chaîne est plus grande que la longueur de fibres des fils du système de fils de trame.

5. Couverture chauffante selon l'une des revendications 2 à 4, caractérisée en ce que le système de fils de trame se compose de fils simples.

6. Couverture chauffante selon l'une des revendications 1 à 5, caractérisée en ce que le système de fils de chaîne du tissu de fibres de carbone est orienté essentiellement transversalement à la longueur de la couverture chauffante (1).

7. Couverture chauffante selon l'une des revendications 1 à 6, caractérisée en ce que les couches flexibles non conductrices d'électricité se composent de silicone.

8. Couverture chauffante selon l'une des revendications 1 à 7, caractérisée en ce que chaque élément de couche chauffante (8) est relié à un dispositif d'alimentation en courant associé.

9. Couverture chauffante selon la revendication 8, caractérisé en ce que pour chaque élément de couche chauffante (8), on a prévu une sonde de température (11) avec régulateur de température associé, qui commande le courant d'alimentation.

10. Couverture chauffante selon l'une des revendications 1 à 9, caractérisée en ce que chaque élément de couche chauffante (8) présente une couche thermo-isolante (4) sur sa face supérieure.

11. Couverture chauffante selon l'une des revendications 1 à 10, caractérisée en ce que la tension d'alimentation de la couche chauffante est de 24 volts ou moins et en particulier de 12 volts environ.

12. Couverture chauffante selon l'une des revendications 1 à 11, caractérisée en ce qu'une chemise ou housse (7) pour la couche chauffante est interchangeable.
